# EUROPEAN PATENT SPECIFICATION

(11) **EP 2 633 860 B1**
(45) Date of publication and mention of the grant of the patent: **08.05.2019**
(21) Application number: 13165012.9
(22) Date of filing: 16.12.2008
(51) Int. Cl.: A61K 35/14, A61K 38/00, A61K 38/48

(54) **Stabilized factor IX formulations containing trehalose**
Stabilisierte Faktor-IX-Formulierungen mit Trehalose
Formulations stabilisées de facteur IX contenant du tréhalose

(30) Priority: 21.12.2007 US 16230 P
(43) Date of publication of application: 04.09.2013
(62) Divisional of application: 08864646.8
(73) Proprietor: Aptevo BioTherapeutics LLC, Seattle WA 98121 (US)
(72) Inventor: Mankarious, Samia, Costa Mesa, CA California 92626 (US); Griffith, Michael J., San Juan Capistrano, CA California 92675 (US)
(74) Representative: Gill Jennings & Every LLP

(56) References cited:
- WO-A1-96/30041
- WO-A1-2006/128497
- WO-A2-2005/123034

## Description

### BACKGROUND OF THE INVENTION

### Field of the Invention

Embodiments of the invention relate to stabilization of Factor IX structure and activity during lyophilization and storage.

### Description of the Related Art

Factor IX is a single-chain glycoprotein that participates in the coagulation pathway. Factor IX is a structurally complex molecule containing an amino terminal signal peptide and prepro leader sequence (both cleaved prior to secretion into circulation) as well as a Gla domain responsible for Ca²⁺ binding. Calcium binding plays an important role in Factor IX function by binding to and inducing a conformational change in the protein that is required for clotting activity. Calcium binding results in exposure of previously buried hydrophobic binding sites that facilitate binding to phospholipids for efficient coagulation. Maintenance of the calcium binding property of Factor IX is necessary in order to produce an active protein. Once the activation peptide has been cleaved, single-chain Factor IX becomes the activated enzyme Factor IXa, a double chain glycoprotein linked via an inter-chain disulfide bond. Additionally, the molecule contains multiple N and O-linked glycosylation sites. Deficiency in Factor IX results in hemophilia B, of which several treatments are currently available, including BeneFIX®, a recombinant version of Factor IX, and Mononine®, which is derived from human plasma.

The formulation of Mononine® consists of histidine, mannitol, sodium chloride and Polysorbate 80. These are largely excipients known to demonstrate eutectic
transitions (crystallization events) during freezing. However, the Mononine® formulation contains no cryoprotectant or stabilizer, only a buffering agent, bulking agent, tonicifier and surfactant. Consequently, during freezing, lyophilization and subsequent storage, the protein is relatively unprotected physically from denaturing effects due to exposure to ice, water and air. The technical problem addressed here is an improved Factor IX formulation, with improved stability during freezing, lyophilization and storage. The present inventors have discovered that inclusion of trehalose in the Factor IX composition during freezing, lyophilization and storage stabilized the calcium binding ability of Factor IX and maintained biological activity of the purified protein.

### SUMMARY OF THE INVENTION

The invention provides lyophilized compositions comprising Factor IX and trehalose, wherein the trehalose is present in an amount from 0.5 to 3% by volume. Yet more preferably, trehalose is present in an amount from 1 to 2% by volume.

In some preferred embodiments, the composition includes histidine as a buffering agent. In some preferred embodiments, the composition includes mannitol. In some preferred embodiments, the composition includes sodium chloride. In some preferred embodiments, the composition includes polysorbate 80.

In a most preferred embodiments, the compositions include Factor IX and trehalose and additionally include histidine at a concentration of 5 to 20 rnM, mannitol at a concentration of 2 to 5% by volume, sodium chloride at a concentration of 50 to 80 mM, and polysorbate 80 at a concentration of 0.001 to 0.005% by volume.

The invention also provides methods for preparing a stable dried composition of Factor IX by mixing a solution containing Factor IX and trehalose to obtain a cryoprotective solution and freeze drying the cryoprotective solution to obtain a stable dried composition of Factor IX, wherein the trehalose is present in an amount from 0.5 to 3% by volume. Yet more preferably, trehalose is present in an amount from 1 to 2% by volume.

In some preferred embodiments, the solution includes histidine as a buffering agent. In some preferred embodiments, the solution includes mannitol. In some preferred embodiments, the solution includes sodium chloride. In some preferred embodiments, the solution includes polysorbate 80.

In a most preferred embodiments, the solution includes Factor IX and trehalose and additionally include histidine at a concentration of 5 to 20 mM, mannitol at a concentration of 2 to 5% by volume, sodium chloride at a concentration of 50 to 80 mM, and polysorbate 80 at a concentration of 0.001 to 0.005% by volume.

Preferably, the freeze drying includes one annealing step.

Further features and advantages of this invention will become apparent from the detailed description of the preferred embodiments which follow.

### BRIEF DESCRIPTION OF THE DRAWINGS

These and other feature of this invention will now be described with reference to the drawings of preferred embodiments which are intended to illustrate and not to limit the invention.

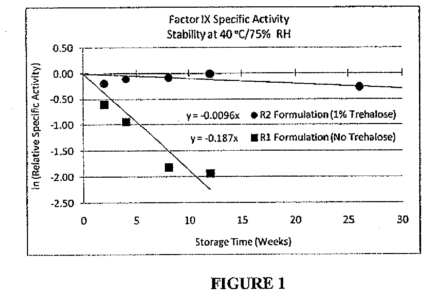

Figure 1 shows Factor IX stability at 40°C/ 75% RH. "■" denotes 0.4mg/ml Factor IX protein in 10mM histidine, 3% mannitol, 66mM NaCl, 0.0075% Polysorbate 80, pH 6.8 (R1). "●" denotes 0.4mg/ml Factor IX protein in 10mM histidine, 3% mannitol, 66mM NaCl, 0.0075% Polysorbate 80, pH 6.8 with 1% trehalose (R2).

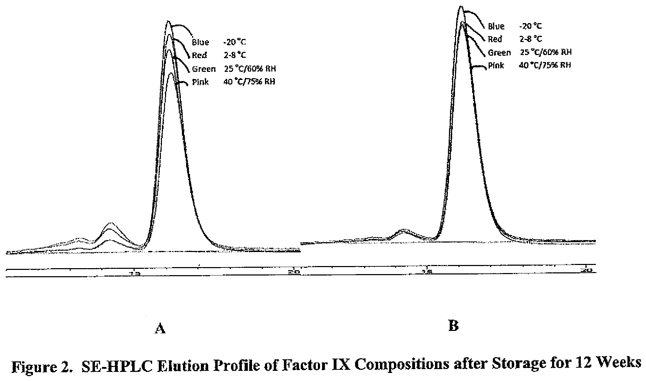

Figure 2 shows the SE-HPLC Elution Profile of Factor IX compositions after Storage for 12 Weeks. Figure 2A shows 0.4mg/ml Factor IX protein in 10mM histidine, 3% mannitol, 66mM NaCl, and 0.0075% Polysorbate 80, pH 6.8 (R1). Figure 2B shows 0.4mg/ml Factor IX protein in 10mM histidine, 3% mannitol, 66mM NaCl, 0.0075% Polysorbate 80, pH 6.8 with 1% trehalose (R2).

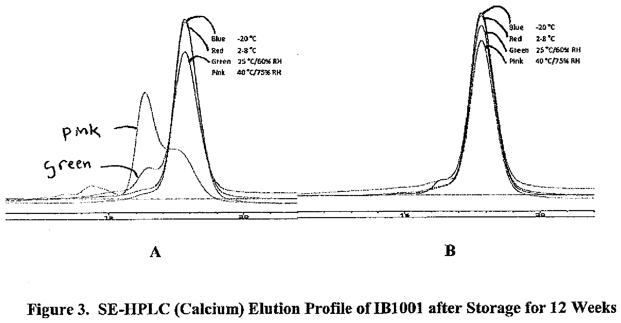

Figure 3 shows SE-HPLC (Calcium) Elution Profile of Factor IX after Storage for 12 Weeks. Figure 3A shows 0.4mg/ml Factor IX protein in 10mM histidine, 3% mannitol, 66mM NaCl, and 0.0075% Polysorbate 80, pH 6.8 (R1). Figure 3B shows 0.4mg/ml Factor IX protein in 10mM histidine, 3% mannitol, 66mM NaCl, 0.0075% Polysorbate 80, pH 6.8 with 1% trehalose (R2).

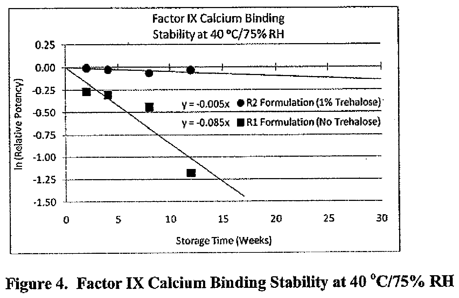

Figure 4 shows Factor IX Calcium Binding Stability at 40 °C/75% RH. "■" denotes 0.4mg/ml Factor IX protein in 10mM histidine, 3% mannitol, 66mM NaCl, 0.0075% Polysorbate 80, pH 6.8 (R1). "●" denotes 0.4mg/ml Factor IX protein in 10mM histidine, 3% mannitol, 66mM NaCl, 0.0075% Polysorbate 80, pH 6.8 with 1% trehalose (R2).

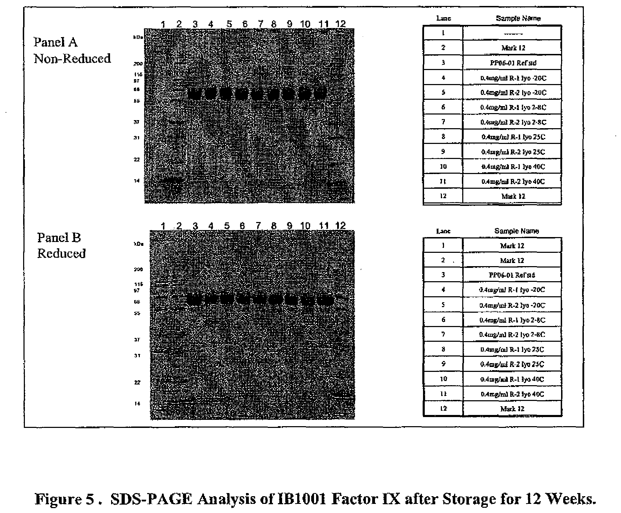

Figure 5 shows SDS-PAGE Analysis of Factor IX formulations after Storage for 12 Weeks under non-reducing (A) and reducing (B) conditions. Panel A: Lane 1, blank; Lane 2, Markers; Lane 3, Factor IX Reference standard (Mononine®); Lane 4, 0.4 mg/ml, R1, lyo -20°C ; Lane 5, 0.4 mg/ml, R2, lyo -20°C ; Lane 6, 0.4 mg/ml, R1, lyo 2-8°C ; Lane 7, 0.4 mg/ml, R2, lyo 2-8°C ; Lane 8, 0.4 mg/ml, R1, lyo 25°C; Lane 9, 0.4 mg/ml, R2, lyo 25°C; Lane 10, 0.4 mg/ml, R1, lyo 40°C; Lane 11, 0.4 mg/ml, R2, lyo 40°C ; Lane 12, Markers. Panel B: Lanes 1 & 2, Markers; Lane 3, Factor IX Reference standard (Mononine®); Lane 4, 0.4 mg/ml, R1, lyo -20°C ; Lane 5, 0.4 mg/ml, R2, lyo -20°C ; Lane 6, 0.4 mg/ml, R1, lyo 2-8°C ; Lane 7, 0.4 mg/ml, R2, lyo 2-8°C ; Lane 8, 0.4 mg/ml, R1, lyo 25°C; Lane 9, 0.4 mg/ml, R2, lyo 25°C; Lane 10, 0.4 mg/ml, R1, lyo 40°C; Lane 11, 0.4 mg/ml, R2_{:} lyo 40°C ; Lane 12, Markers.

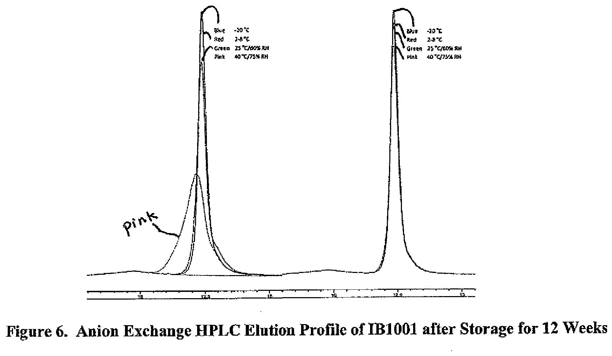

Figure 6 shows Anion Exchange HPLC Elution Profile of Factor IX compositions after Storage for 12 Weeks. Figure 6A shows 0.4mg/ml Factor IX protein in 10mM histidine, 3% mannitol, 66mM NaCl, and 0.0075% Polysorbate 80, pH 6.8 (R1). Figure 6B shows 0.4mg/ml Factor IX protein in 10mM histidine, 3% mannitol, 66mM NaCl, 0.0075% Polysorbate 80, pH 6.8 with 1% trehalose (R2).

### DETAILED DESCRIPTION OF THE PREFERRED EMBODIMENT

Embodiments of the invention are directed to methods of lyophilizing Factor IX in the presence of trehalose and Factor IX formulations containing trehalose. Factor IX formulations were evaluated with and without the addition of trehalose, a cryoprotectant that does not crystallize during the lyophilization process. Trehalose persists as a concentrate that undergoes glass transition with freezing to lower temperatures. Protein is thus preserved in the presence of the trehalose, retained in a mixed amorphous state and stabilized by freezing and freeze drying. Trehalose improved stability and prevented aggregation of the stored Factor IX protein. It was also found surprisingly that the inclusion of trehalose in the formulation significantly improved preservation of the calcium induced conformational change required for Factor IX activity. Apparent loss of calcium binding ability correlated with loss of potency. This loss in calcium binding ability and potency was greatly retarded in formulations that included trehalose.

In preferred embodiments, at least 50%, more preferably at least 70%, yet more preferably at least 80%, yet more preferably at least 90% of the calcium binding capability of Factor IX is preserved by inclusion of trehalose in the Factor IX composition during freezing, lyophilization and storage. Preferably, the trehalose is present in an amount sufficient to preserve more than 90% of the calcium binding property of Factor IX during freezing, lyophilization and storage for at least three months at 25°C, more preferably at least 6 months at 25°C and yet more preferably at least 1 year at 25°C.

### Formulation Components

In preferred embodiments, the Factor IX compositions of the present invention include a buffering agent, a bulking agent, tonicifier, surfactant and cryoprotectant/stabilizer. In some embodiments, other excipients may also be included. These compositions maximize the stability of Factor IX in lyophilized preparations and in the liquid state as well.

In preferred embodiments, a buffering agent is included in the composition. The pH should preferably be maintained in the range of between 6 and 8 during lyophilization and storage, and more preferably at a pH of about 6.8. The buffering agent can be any physiologically acceptable chemical entity or combination of chemical entities which have the capacity to act as buffers, including histidine, tris-(hydroxymethyl)-aminomethane (TRIS), 1,3-bis-[tris-(hydroxy-methyl)methylamino]-propane (BIS-Tris Propane), piperazine-N,N'-bis-(2-ethanesulfonic acid) (PIPES), 3-(N-morpholino) propanesulfonic acid (MOPS), N-2-hydroxyethyl-piperazine-N'-2-ethanesulfonic acid (HEPES), 2-(N-morpholino) ethanesulfonic acid (MES) and N-2-acetamido-2-aminoethanesulfonic acid (ACES). Typically, the buffering agent is included in a concentration of 5-20 mM. In a most preferred embodiment, the buffering agent is histidine at a concentration of about 10 mM.

Bulking agents are those chemical entities which provide structure to the "cake" or residual solid mass of a pharmaceutical preparation after it has been lyophilized and which protect it against collapse. The bulking agents used in the present formulations are selected from the group including but not limited to mannitol, glycine, and alanine. Mannitol, glycine, or alanine are present in an amount of 1-10%, preferably 2-5%, and more preferably about 3%.

Sodium chloride is included in the present formulations in an amount of 30-100 mM, preferably 50-80 mM, and most preferably about 66 mM.

In preferred embodiments, the Factor IX compositions include a surfactant, preferably in an amount of 0.1% or less, and more preferably in an amount of 0.001-0.005%. The surfactant can, for example, be selected from the group including but not limited to polysorbate 20, polysorbate 80, pluronic polyols, and BRIJ 35 (polyoxyethylene 23 lauryl ether). Several grades of pluronic polyols (sold under the trade name PLURONIC, manufactured by the BASF Wyandotte Corporation) are available. These polyols, of diversified molecular weight (from 1,000 to over 16,000) and physicochemical properties have been used as surfactants. PLURONIC F-38, of a molecular weight of 5,000 and PLURONIC F-68, molecular weight 9,000, both contain (by weight) 80 per cent hydrophilic polyoxyethylene groups and 20 percent hydrophobic polyoxypropylene groups. In preferred embodiments, Polysorbate 80 is included at a concentration of about 0.0075%.

Preferably, a stabilizing agent is used in the formulations of the present invention. The stabilizer is selected from the group including but not limited to sucrose, trehalose, raffinose, and arginine. These agents are present in the formulations of the present invention in an amount of between 0.5-3%, preferably 1-2%, more preferably about 1%. In a highly preferred embodiment, trehalose is included in the composition at a concentration of 1%.

In preferred embodiments, the Factor IX used in the present compositions is either highly purified human plasma-derived Factor IX or more preferably can be recombinantly produced Factor IX. Recombinant Factor IX can be produced by Chinese hamster ovary (CHO) cells transfected with a vector carrying a DNA sequence coding for the Factor IX molecule. Methods for creating such transfected CHO cells are described, inter alia, in U.S. Pat. No. 4,757,006 to Toole, Jr., though alternative methods are also known to the art (see, e.g., U.S. Pat. No. 4,868,112, also to Toole, Jr., and PCT International Application WO-A-91/09122).

While the Factor IX compositions described in this application can be lyophilized and reconstituted in the indicated concentrations, one of skill in the art will understand that these preparations can also be reconstituted in more dilute form. For example, a preparation according to the present invention which is lyophilized and/or normally reconstituted in 2 ml of solution can also be reconstituted in a larger volume of diluent, such as 5 ml. This is particularly appropriate when the Factor IX preparation is being injected into a patient immediately, since in this case the Factor IX is less likely to lose activity, which may occur more rapidly in more dilute solutions of Factor IX.

### EXAMPLES

Recombinant Factor IX was prepared in Chinese hamster ovary cells transfected with cDNA encoding human Factor IX. Factor IX was purified from conditioned media using a process including anion and cation exchange chromatography to separate the desired product from media components including host cell proteins and DNA.

Lyophilization was carried out by means known in the art. Information on lyophilization may be found in Carpenter, J. F. and Chang, B. S., Lyophilization of Protein Pharmaceuticals, Biotechnology and Biopharmaceutical Manufacturing, Processing and Preservation, K. E. Avis and V. L. Wu, eds. (Buffalo Grove, Ill.: Interpharm Press, Inc.), pp. 199-264 (1996). In the context of the present invention, the terms "freeze drying" and "lyophilization" are used interchageably to include all of the steps for concentrating the sample, including annealing and drying steps. In preferred embodiments, the lyophilization includes 1-3 annealing steps. In preferred embodiments, lyophilization is carried out with one annealing step. The term "anneal" indicates a step in the lyophilization process of a pharmaceutical preparation undergoing lyophilization, prior to the freeze-drying of the preparation, in which the temperature of the preparation is raised from a lower temperature to a higher temperature and then cooled again after a period of time. The drying steps are carried out under reduced pressure, typically in the range of 50-300 microbar.

An exemplary protocol is illustrated below in Table 1.

**TABLE 1**

| **Step #** | **Step** | **Shelf Temperature °C** | **N2 Bleeding** | **Time (Minutes)** | **Pressure (microbar)** |
|---|---|---|---|---|---|
| 1 | Loading | Room Temperature | | | Ambient |
| 2 | Freezing | -50 | | 100 | Ambient |
| 3 | Freezing | -50 | | 120 | Ambient |
| 4 | Freezing | -23 | | 60 | Ambient |
| 5 | Freezing | -23 | | 120 | Ambient |
| 6 | Freezing | -50 | | 60 | Ambient |
| 7 | Freezing | -50 | | 120 | Ambient |
| 8 | Primary Drying | -50 | X | 60 | 150 |
| 9 | Primary Drying | -43 | X | 100 | 150 |
| 10 | Primary Drying | 10 | X | 120 | 150 |
| 11 | Primary Drying | 10 | X | 1440 | 150 |
| 13 | Secondary Drying | 50 | X | 435 | 150 |
| 14 | Secondary Drying | 50 | X | 240 | 150 |
| 15 | Pre aeration | 25 | X | | 150 |
| 16 | Stoppering | 25 | X | 0.15 | 150 |
| 17 | Storage | 4 | X | < 72 hours | < 10⁶ |
| 18 | Aeration | 4 | X | | Ambient |

Activity was determined with the Factor IX one-stage clotting assay. One stage assays are known in the art. The assay used here utilizes a Universal Coagulation Reference Plasma (UCRP) as a standard for Factor IX activity and Factor IX-deficient plasma for dilution of calibration standards and unknown samples. The assay involves mixing plasma with activator and calcium chloride to initiate the clotting cascade, with formation of the fibrin clot measured by absorbance on a microplate reader. The clotting time measured in this assay is the aPTT (activated partial thromboplastin time), the time required for the absorbance to cross a pre-determined threshold value. Accurate determination of Factor IX activity is achieved by comparing the signal of the unknowns to Factor IX Reference Standard (UCRP) assayed simultaneously. Note that all data presented are from 1 vial per temperature per time point.

### EXAMPLE 1

### Factor IX in a stabilized formulation containing trehalose shows increased stability during storage at 25°C and 40°C.

Factor IX was lyophilized in each of the two candidate formulations as shown in Table 2 below. Both formulations included 10 mM histidine, 3% mannitol, 66 mM NaCl, 0.0075% Polysorbate 80, pH 6.8. One of the formulations (R2) additionally contained trehalose (1%). The formulations were evaluated over 26 weeks at real time storage conditions of -20°C and 2-8°C as well as conditions of 25°C/60% RH and 40°C/75% RH. Factor IX at 0.4 mg/mL was evaluated throughout the study by a panel of analytical methods including Size exclusion (SE)-HPLC, Ion exchange (IE)-HPLC, Reverse Phase (RP)-HPLC, SDS-PAGE, protein concentration, turbidity, pH, visual appearance (cake and reconstituted liquid) residual moisture and activity. The formulation configuration was a 5 mL fill in 10 mL glass vials.

The assay results obtained at each time point were normalized by dividing the measured values for each storage condition by the measured value obtained for the formulated product stored at -20 °C which was, by definition, taken to represent 100%. This approach was adopted in an effort to minimize research laboratory assay variability during the time period of the study.

**TABLE 2**

| **STABILITY EVALUATION TIME POINTS** | | | | | | | |
|---|---|---|---|---|---|---|---|
| | | Weeks | | | | | |
| Factor IX Formulation | Storage Condition | 0 | 2 | 4 | 8 | 12 | 26 |
| 10 mM histidine, 3% mannitol, 66 mM NaCl, 0.0075% Polysorbate 80, pH 6.8 | -20°C | X | X | X | X | X | X |
| | 4°C | | | X | X | X | X |
| | 25°C/60%RH | | X | X | X | X | |
| | 40°C/75%RH | | X | X | X | X | |
| 10 mM histidine, 3% mannitol, 1% trehalose, 66 mM NaCl, 0.0075% Polysorbate 80, pH 6.8 | -20°C | X | X | X | X | X | X |
| | 4°C | | | X | X | X | X |
| | 25°C/60%RH | | X | X | X | X | |
| | 40°C/75%RH | | X | X | X | X | |

**TABLE 3**

| Formulation Without Trehalose | | | | | | | | | | |
|---|---|---|---|---|---|---|---|---|---|---|
| Storage Condition | Rate Constant | Storage Time | % Functional | | Storage Time | % Functional | | Storage Time | % Functional | |
| | | | Calculated | Measured | | Calculated | Measured | | Calculated | Measured |
| 2-8 ?C | 0.00198 | 12 | 102% | 106% | 26 | 105% | 102% | 52 | 111% | n.d. |
| 25 ?C | -0.01960 | 12 | 79% | 74% | 26 | 60% | n.d. | 52 | 36% | n.d. |
| 40 ?C | -0.18700 | 12 | 11% | 15% | 26 | 1% | n.d. | 52 | 0% | n.d. |

| Formulation With Trehalose | | | | | | | | | | |
|---|---|---|---|---|---|---|---|---|---|---|
| Storage Condition | Rate Constant | Storage Time | % Functional | | Storage Time | % Functional | | Storage Time | % Functional | |
| | | | Calculated | Measured | | Calculated | Measured | | Calculated | Measured |
| 2-8 ?C | 0.00044 | 12 | 101% | 97% | 26 | 101% | 102% | 52 | 102% | n.d. |
| 25 ?C | -0.00390 | 12 | 95% | 97% | 26 | 90% | 90% | 52 | 82% | n.d. |
| 40 ?C | -0.00960 | 12 | 89% | 99% | 26 | 78% | 76% | 52 | 61% | n.d. |

As indicated by the results shown in Table 3, the addition of trehalose (1%) dramatically improved the stability of lyophilized Factor IX during storage at 40 °C/75% RH. Whereas the specific activity, i.e. Factor IX activity units/mg of protein in reconstituted drug product, of formulation without trehalose decreased to ∼15% during 12 weeks of storage at 40 °C/75% RH, the specific activity of Factor IX formulated with trehalose was only modestly reduced.

As shown in Figure 1, an analysis of the rate of decay of specific activity over the 12 week time period suggests that trehalose decreases the rate of decay at 40 °C/75% RH by almost 20-fold (-0.0096 wk⁻¹ vs -0.187 wk⁻¹).

The protective effect of trehalose on the stability of lyophilized Factor IX stored under room temperature conditions (nominally 25 °C/60%) is also noteworthy. The apparent effect of trehalose is to reduce the rate of decay of specific activity by ∼5-fold, from 0.0196 wk⁻¹ to 0.0039 wk⁻¹ (Table 3). The apparent rate of decay of Factor IX formulated with trehalose indicates that the lyophilized product would be stable when stored at room temperature for up to 26 weeks (6 months). Both measured and calculated specific activity values provide support that trehalose formulated Factor IX may retain ∼90% activity at 26 weeks (Table 3).

### EXAMPLE 2

### Factor IX formulations containing trehalose show less aggregation during storage of lyophilized product as shown by size exclusion HPLC (SE-HPLC)

The formation of high molecular weight aggregates, detected by size exclusion chromatography (SE-HPLC), decreases the apparent purity and specific activity of Factor IX preparations. The addition of trehalose (1%) to the R1 formulation buffer appears to substantially prevent Factor IX aggregation during storage of the lyophilized product. The SE-HPLC elution profiles for R1 and R2 formulated products after 12 weeks storage are shown in Figure 2.

Size exclusion of Factor IX was performed using a Tosoh G3000SWxl column (7.8 mm x 30 cm, 5 µm, 250 Å) on Agilent 1100 series HPLC's. The isocratic method employed 50 mM Tris, 200 mM NaCl pH 7.5 as the mobile phase.

### EXAMPLE 3

### Size exclusion HPLC in the presence of calcium shows that Factor IX stored with trehalose maintains ability to undergo calcium induced conformational change.

Calcium ions play an important role in Factor IX function by binding to and inducing a conformational change in the protein that is required for clotting activity. The calcium-induced conformational change, which decreases the hydrodynamic volume of the protein in solution, can be detected as a decrease in the apparent molecular weight by methods such as SE-HPLC.

As was observed when Factor IX activity was directly measured (Table 3), the addition of trehalose (1%) to the formulation buffer dramatically improves the stability of lyophilized Factor IX during storage in terms of preserving Factor IX function which, in this case, is the ability to bind calcium and undergo the calcium-induced conformational change. The SE-HPLC elution profiles, in the presence of calcium, for Factor IX compositions in the presence (R2) and absence (R1) of trehalose after 12 weeks storage are shown in Figure 3. Whereas the percentage of functional Factor IX in R1 formulated drug product (without trehalose) decreased to ∼31% during 12 weeks of storage at 40 °C/75% RH, the percentage functional Factor IX in R2 formulated drug product (+ trehalose) was only slightly lower than the Factor IX stored at low temperatures (-20°C, 2-8 °C).

As shown in Figure 4, an analysis of the rate of decay in function (calcium-induced conformational change) over the 12 week time period suggests that trehalose decreases the rate of decay at 40 °C/75% RH by ∼19-fold (-0.0046 wk⁻¹ vs -0.0853 wk⁻¹), which is very similar to the ∼20-fold decrease in the rate of decay in potency (Figure 1).

### EXAMPLE 4

### Factor IX formulated with trehalose shows less contamination by high molecular weight contaminants

SDS-PAGE was performed to obtain a direct visual comparison of the purity of lyophilized Factor IX after storage for 12 weeks under the various conditions. As shown in Panel A of Figure 5, trace amounts of high molecular contaminants appear to be present in all samples, but the amount is progressively greater for Factor IX that has been stored at higher temperatures. This is most readily seen in lanes 8 and 10 of the non-reduced SDS-PAGE gel where samples from R1 formulated Factor IX (no trehalose) that was held at 25 °C/60% RH and 40 °C/75% RH, respectively are shown. The corresponding samples from R2 (plus trehalose) formulated Factor IX, shown in lanes 9 and 11, show little evidence for an increase in the amount of high molecular weight contaminants when compared to samples of either R1 or R2 formulated Factor IX that was stored at 2-8 °C or -20 °C (lanes 4 - 7).

While degraded forms of Factor IX were detectable in the preparation of Factor IX that was used in the present study, the amounts did not appear to increase with storage time under any of the experimental conditions. Factor IX-gamma (Factor IXγ) is a truncated, lower molecular weight form of Factor IX that is formed when the intact protein is proteolytically cleaved at or near the Arg318-Ser319 peptide bond to release a 10 kDa peptide from the carboxy-terminal region of the molecule. The Factor IXγ that is present in the Factor IX formulations can be seen in non-reduced SDS-PAGE gels (Panel A) as a minor band that migrates with an apparent molecular weight of approximately 45 kDa. Visual inspection of the gel shown in Panel A of Figure 5 suggests that significant proteolysis of Factor IX to Factor IXγ has not occurred during storage over the time period of the present study.

### EXAMPLE 5

### Ion exchange chromatography shows that trehalose stabilized Factor IX compositions.

Ion exchange chromatography has the potential to partially separate protein isoforms that differ in charge and/or charge distribution. Anion exchange chromatography of Factor IX was performed using a GE Healthcare Tricorn MonoQ 5/50GL column (5 x 50 mm, 10 µm). The binary gradient method utilized 50 mM Tris pH 7.5 as mobile phase A, and 50 mM Tris, 1 M NaCl pH 7.5 as mobile phase B.

Anion exchange chromatography, as performed in the present study, resulted in the elution of R1 formulated Factor IX (no trehalose) as a single symmetrical peak that broadened over storage time at 40 °C/75% RH, whereas the elution of R2 formulated Factor IX (plus trehalose) appeared to be substantially unchanged in this regard. These results are shown in Figure 6.

### Conclusion

By adding trehalose (1%) to a formulation known to stabilize highly purified lyophilized Factor IX for at least two years at 2-8 °C, an even better formulation is obtained in terms of maintaining protein structure and function. Factor IX composition without trehalose (R1) appeared to be stable at room temperature (25 °C/60% RH) for approximately one month. In the presence of trehalose (R2), Factor IX drug product was stable for approximately six months (based on >90% retained activity).

When Factor IX is formulated with and without trehalose in a lyophilized formulation containing histidine, mannitol, sodium chloride and Polysorbate 80, the formulation with trehalose presents a superior stability profile during storage at 25°C and 40°C, likely due to the cryoprotective benefit produced by the amorphous properties of the dried disaccharide.

The Factor IX formulation with trehalose presented stability data comparable to that of Factor IX stored at refrigerated temperatures. The data support the possibility for temperature excursions of Factor IX drug product in the formulation with trehalose at room temperature for several weeks and potentially even longer.

Storage of Factor IX in the formulation without trehalose at 40°C/75%RH led to:
- an increase in high molecular weight species as identified by SE-HPLC.
- a trend of decreasing activity over 12 weeks as determined by the one-stage clotting assay.
- a significant broadening of the IE-HPLC chromatographic profile.

Storage of Factor IX in the formulation without trehalose at 25°C/60%RH also resulted in degradation as described above, although to a lesser extent.

No significant differences were observed between the formulations with respect to cake morphology, concentration, turbidity of the reconstituted product or RP-HPLC.

26 week stability of Factor IX at refrigerated and frozen temperatures were comparable for both formulations.

Residual moisture levels and reconstitution times were each slightly higher for the formulation with trehalose as compared to the formulation without.

### FURTHER EMBODIMENTS

1. A lyophilized composition, comprising Factor IX and trehalose, wherein the trehalose is present in an amount sufficient to preserve more than 90% of the calcium binding property of Factor IX during lyophilization and storage for 6 months at 25°C.
2. The composition of claim 1, wherein trehalose is present in an amount from 0.5 to 3% by volume.
3. The composition of claim 2, wherein trehalose is present in an amount from 1 to 2% by volume.
4. The composition of any one of claims 1-3, further comprising histidine as a buffering agent.
5. The composition of any one of claims 1-3, further comprising mannitol.
6. The composition of any one of claims 1-3, further comprising sodium chloride.
7. The composition of any one of claims 1-3, further comprising polysorbate 80.
8. The composition of any one of claims 1-3, further comprising histidine at a concentration of 5 to 20 mM, mannitol at a concentration of 2 to 5% by volume, sodium chloride at a concentration of 50 to 80 mM, and polysorbate 80 at a concentration of 0.001 to 0.005% by volume.
9. A method for preparing a stable dried composition of Factor IX, comprising:
   mixing a solution comprising Factor IX with trehalose to obtain a cryoprotective solution; and
   freeze drying the cryoprotective solution to obtain a stable dried composition of Factor IX
      wherein the dried composition of Factor IX retains more than 90% calcium binding activity when stored for 6 months at 25°C.
10. The method of claim 9, wherein trehalose is present in an amount from 0.5 to 3% by volume.
11. The method of claim 10, wherein trehalose is present in an amount from 1 to 2% by volume.
12. The method of any one of claims 9-11, wherein the solution further comprises histidine.
13. The method of any one of claims 9-11, wherein the solution further comprises mannitol.
14. The method of any one of claims 9-11, wherein the solution further comprises sodium chloride.
15. The method of any one of claims 9-11, wherein the solution further comprises polysorbate 80.
16. The method of any one of claims 9-11, wherein the solution further comprises histidine at a concentration of 5 to 20 mM, mannitol at a concentration of 2 to 5% by volume, sodium chloride at a concentration of 50 to 80 mM, and polysorbate 80 at a concentration of 0.001 to 0.005% by volume.
17. The method of any one of claims 9-11, wherein the freeze drying comprises one annealing step.
18. A method of lyophilizing a pharmaceutical formulation comprising Factor IX and trehalose comprising the steps of :
   (a) freezing the pharmaceutical formulation comprising Factor IX and trehalose at a temperature of -40°C or less;
   (b) annealing the pharmaceutical formulation at between about -20°C and - 35°C;
   (c) lowering the temperature of the pharmaceutical formulation to -40°C or less;
   (d) drying the pharmaceutical formulation in a first drying step at 5°C to 20°C at reduced pressure; and
   (e) drying the pharmaceutical formulation in a second drying step at 45°C to 55°C at reduced pressure.
19. The method of claim 18, wherein the pharmaceutical formulation further comprises histidine at a concentration of 5 to 20 mM, mannitol at a concentration of 2 to 5% by volume, sodium chloride at a concentration of 50 to 80 mM, and polysorbate 80 at a concentration of 0.001 to 0.005% by volume.

## Claims

1. A lyophilized composition comprising Factor IX and trehalose, wherein the trehalose is present in an amount from 0.5 to 3% by volume.

2. A composition according to claim 1 wherein, before lyophilization, trehalose is present in an amount from 1 to 2%.

3. A composition according to any of claims 1 or 2, further comprising histidine.

4. A composition according to any of claims 1 to 3, further comprising mannitol, sodium chloride and/or polysorbate 80.

5. A composition according to any of claims 1 to 4, wherein, before lyophilization, the composition further comprises histidine at a concentration of 5 to 20 mM, mannitol at a concentration of 2 to 5%, sodium chloride at a concentration of 50 to 80 mM, and polysorbate 80 at a concentration of 0.001 to 0.005% by volume.

6. A method for preparing a composition of Factor IX, comprising:
mixing a solution comprising Factor IX with trehalose to obtain a cryoprotective solution; and
freeze drying the cryoprotective solution to obtain a composition of Factor IX, wherein the trehalose is present in an amount from 0.5 to 3% by volume.

7. A method according to claim 6, wherein trehalose is present in an amount from 1% to 2% in the cryoprotective solution.

8. A method according to any of claims 6 or 7, wherein the solution further comprises histidine, mannitol, sodium chloride and/or polysorbate 80.

9. A method according to any of claim 6 to 8, wherein the solution further comprises histidine at a concentration of 5 to 20 mM, mannitol at a concentration of 2 to 5%, sodium chloride at a concentration of 50 to 80 mM, and polysorbate 80 at a concentration of 0.001 to 0.005% by volume.

10. A method according to any of claims 6 to 9, wherein the freeze drying comprises one annealing step.

## Patentansprüche

1. Lyophilisierte Zusammensetzung, die Faktor IX und Trehalose umfasst, wobei die Trehalose in einer Menge von 0,5 bis 3 Volumen-% vorliegt.

2. Zusammensetzung nach Anspruch 1, wobei die Trehalose vor der Lyophilisierung in einer Menge von 1 bis 2 % vorliegt.

3. Zusammensetzung nach einem der Ansprüche 1 oder 2, die weiter Histidin umfasst.

4. Zusammensetzung nach einem der Ansprüche 1 bis 3, die weiter Mannitol, Natriumchlorid und/oder Polysorbat 80 umfasst.

5. Zusammensetzung nach einem der Ansprüche 1 bis 4, wobei die Zusammensetzung vor der Lyophilisierung weiter Histidin in einer Konzentration von 5 bis 20 mM, Mannitol in einer Konzentration von 2 bis 5 %, Natriumchlorid in einer Konzentration von 50 bis 80 mM und Polysorbat 80 in einer Konzentration von 0,001 bis 0,005 Volumen-% umfasst.

6. Verfahren für die Herstellung einer Zusammensetzung von Faktor IX, das Folgendes umfasst:
Mischen einer Lösung, die Faktor IX umfasst, mit Trehalose, um eine kryoprotektive Lösung zu erhalten;
und
Gefriertrocknen der kryoprotektiven Lösung, um eine Zusammensetzung von Faktor IX zu erhalten, wobei die Trehalose in einer Menge von 0,5 bis 3 Volumen-% vorliegt.

7. Verfahren nach Anspruch 6, wobei Trehalose in einer Menge von 1 % bis 2 % in der kryoprotektiven Lösung vorliegt.

8. Verfahren nach einem der Ansprüche 6 oder 7, wobei die Lösung weiter Histidin, Mannitol, Natriumchlorid und/oder Polysorbat 80 umfasst.

9. Verfahren nach einem der Ansprüche 6 bis 8, wobei die Lösung weiter Histidin in einer Konzentration von 5 bis 20 mM, Mannitol in einer Konzentration von 2 bis 5 %, Natriumchlorid in einer Konzentration von 50 bis 80 mM und Polysorbat 80 in einer Konzentration von 0,001 bis 0,005 Volumen-% umfasst.

10. Verfahren nach einem der Ansprüche 6 bis 9, wobei das Gefriertrocknen einen Temperschritt umfasst.

## Revendications

1. Composition lyophilisée comprenant du Facteur IX et du tréhalose, dans laquelle le tréhalose est présent en une quantité de 0,5 à 3 % en volume.

2. Composition selon la revendication 1, dans laquelle, préalablement à la lyophilisation, le tréhalose est présent en une quantité de 1 à 2 %.

3. Composition selon l'une quelconque des revendications 1 ou 2, comprenant en outre de l'histidine.

4. Composition selon l'une quelconque des revendications 1 à 3, comprenant en outre du mannitol, du chlorure de sodium et/ou du polysorbate 80.

5. Composition selon l'une quelconque des revendications 1 à 4, dans laquelle préalablement à la lyophilisation, la composition comprend en outre de l'histidine à une concentration de 5 à 20 mM, du mannitol à une concentration de 2 à 5 %, du chlorure de sodium à une concentration de 50 à 80 mM et du polysorbate 80 à une concentration de 0,001 à 0,005 % en volume.

6. Procédé de préparation d'une composition de Facteur IX, comprenant :
mélanger une solution comprenant du Facteur IX avec du tréhalose pour obtenir une solution cryoprotectrice ; et
lyophiliser la solution cryoprotectrice pour obtenir une composition de Facteur IX, dans laquelle le tréhalose est présent en une quantité de 0,5 à 3 % en volume.

7. Procédé selon la revendication 6, dans lequel le tréhalose est présent en une quantité de 1 % à 2 % dans la solution cryoprotectrice.

8. Procédé selon l'une quelconque des revendications 6 ou 7, dans lequel la solution comprend en outre de l'histidine, du mannitol, du chlorure de sodium et/ou du polysorbate 80.

9. Procédé selon l'une quelconque des revendications 6 à 8, dans lequel la solution comprend en outre de l'histidine à une concentration de 5 à 20 mM, du mannitol à une concentration de 2 à 5 %, du chlorure de sodium à une concentration de 50 à 80 mM et du polysorbate 80 à une concentration de 0,001 à 0,005 % en volume.

10. Procédé selon l'une quelconque des revendications 6 à 9, dans lequel la lyophilisation comprend une étape de recuit.
